# EUROPEAN PATENT SPECIFICATION

(11) **EP 1 276 891 B1**
(45) Date of publication and mention of the grant of the patent: **18.05.2005**
(21) Application number: 00940731.3
(22) Date of filing: 29.03.2000
(51) Int. Cl.: C12P 7/64, C12N 1/10, C12N 1/38

(54) **A METHOD FOR ENHANCING LEVELS OF POLYUNSATURATED FATTY ACIDS IN THRAUSTOCHYTRIDS**
VERFAHREN ZUR ERHÖHUNG VON MEHRFACHUNGESÄTTIGTEN FETTSÄUREN IN THRAUSTOCHYTRIDEN
METHODE PERMETTANT D'ACCROITRE LES TAUX D'ACIDES GRAS INSATURES DANS DES CHAMPIGNONS DU TYPE i THRAUSTOCHYTRIUM /i

(43) Date of publication of application: 22.01.2003
(73) Proprietor: Council of Scientific and Industrial Research, New Delhi 110 001 (IN)
(72) Inventor: KUMAR, Seshagiri Raghu, Dona Paula, Goa 403 004 (IN); CHANDRAMOHAN, Dorai Rajasingam, Dona Paula, Goa 403 004 (IN); DESA, Ehrlich, Dona Paula, Goa 403 004 (IN)
(74) Representative: Evans, Claire
(86) International application number: PCT/IN2000/000037
(87) International publication number: WO 2001/073099

(56) References cited:
- EP-A- 0 207 475
- WO-A-98/03671
- BOWLES R D ET AL: "Long-chain n-3 polyunsaturated fatty acid production by members of the marine protistan group the thraustochytrids: screening of isolates and optimisation of docosahexaenoic acid production" JOURNAL OF BIOTECHNOLOGY,NL,ELSEVIER SCIENCE PUBLISHERS, AMSTERDAM, vol. 70, no. 1-3, 30 April 1999 (1999-04-30), pages 193-202, XP004173399 ISSN: 0168-1656

## Description

### FILED OF THE INVENTION

This present invention relates to a method for enhancing levels of polyunsaturated fatty acids in thraustochytrid fungi. The present invention particularly relates to a process for enhancement of the polyunsaturated fatty acids, docosahexaenoic acid and eicosapentaenoic acid in cells of microorganisms belonging to the group of fungi termed thraustochytrids, by growing the cells in a medium with increased viscosity. The cells thus enriched in the said polyunsaturated fatty acids (PUFAs) can then be utilized more successfully than cells that are not enriched in the PUFAs, in various beneficial applications that require polyunsaturated fatty acids, such as in animal feeds, human nutrition and extraction of the PUFAs for nutritional supplementation.

### BACKGROUND OF THE INVENTION

Fatty acids are constituents of lipids, which are required by all living organisms for growth, survival and reproduction. Among the fatty acids, saturated fatty acids are those with a chemical structure in which the carbon atoms are connected to each other only by single bonds and contain no double bonds. Unsaturated fatty acids are those in which one or more of the carbon atoms are connected to each other by double bonds. Polyunsaturated fatty acids, termed as PUFAs hereafter, are those in which more than one such double bonds are found.

Among the PUFAs, two are considered extremely essential in the health of animals and human beings. These are the docosahexaenoic acid and eicosapentaenoic acid, termed DHA and EPA hereafter. The molecular structure of both DHA and EPA is such that the first double bond follows the third carbon atom from the methyl end of the fatty acid structure. Therefore, these are also called omega-3 PUFAs. DHA contains 22 carbon atoms, between which six double bonds are found. EPA contains 20 carbon atoms, between which five double bonds occur. Both DHA and EPA have been shown to be important for human health and in animal nutrition. In human health, DHA and EPA have been shown to be important are found. EPA contains 20 carbon atoms, between which five double bonds occur. Both DHA and EPA have been shown to be important for human health and in animal nutrition. In human health, DHA and EPA have been shown to be important in brain development in children, prevention of atherosclerosis, prevention of night blindness, neurological disorders and even for possible prevention of cancer (Bajpai, P. and P.K. Bajpai. 1993. Journal of Biotechnology 30: 161-183; Barclay, W.R et al. 1994. Journal of Applied Phycology 6: 123-129; US Patent No. 942 8913; Singh, A. and O.P. Ward. 1997. Advances in applied microbiology, 45: 271-312). These two omega-3 PUFAs have been shown to enhance growth and reproduction in crustacean animals, such as prawns, which are very important as aquaculture animals for human consumption (Harrison, K.E. 1990. Journal of Shellfish Research 9: 1-28). Incorporation of DHA and EPA in human and animal feeds is therefore considered important. DHA and EPA levels of thraustochytrid fungi can be enhanced beyond their natural levels by growing the cells in a medium with increased viscosity, as detailed in the present invention, and their cells can be of still better use as supplement to human nutrition and as feed for animals compared to presently known processes. Thraustochytrids can be cultivated on a large scale, using well established fermentation techniques. Cells thus obtained can be used as animal feeds, by suitably processing and preserving their cells, such as by spray-drying and freezing. The cell biomass, enhanced in the omega-3 fatty acids can also be harvested and DHA and EPA extracted in a pure form. These may be used to supplement human food that is poor in these essential omega-3 PUFAs.

One major source of EPA and DHA for human consumption is in the form of fish oil. However, fish oil has the disadvantage of an odour, which is disagreeable to many human consumers. Fish containing DHA and EPA are also highly seasonal and variable in their omega-3 PUFA contents. Besides, most of the fish oil is hydrogenated and the omega-3 PUFAs are destroyed. For these reasons, microorganisms containing EPA and DHA, which can be cultivated on a large scale are considered suitable for use in human nutrition and animal feeds (Bajpai, P. and P.K. Bajpai.1993. Journal of Biotechnology 30: 161-183). Several single-celled plants, the algae, contain high levels of EPA and DHA and have been considered for the said purposes. Reference may be made to D.L. Alonso et al. (Alonso, D.L. et al., 1992. Aquaculture 102: 363-371). However, large scale cultivation of these plants in natural ponds often is subject to the problem of other microorganisms growing along with these plants. This may pose a health problem to human consumers. Growing them in pure cultures in fermentors is cost-intensive, since these plants require light and suitable photo reactors are very expensive to maintain and operate. Microorganisms can be easily cultivated on a large scale using cheap nutrients. Several groups of microorganisms contain high amounts of EPA and DHA. Such organisms can be used directly as feed, or the said PUFAs can be extracted from them for further use. WO98/03671 relates to a process which includes the steps of cultivating a microorganism which belongs to the genus Ulkenia having the ability to produce DHA and/or DPA. Search for microorganisms containing high amounts of DHA and EPA has shown that thraustochytrid fungi contain some of the highest amounts of DHA and EPA. Thraustochytrids are already considered of commercial importance. Their cells are used in animal feeds or for extraction of PUFAs for commercial use (Singh, A. and O.P. Ward. 1997. Advances in applied Microbiology 45: 271-312). The Japanese Patent No. 9633263 (1996) describes a strain of a thraustochytrid for application in the food industry such as food-additives, nutritional supplements, as additives for infant milk formula, feedstuffs and drug additives. The strain contains at least 2 % of dry wt as DHA. Another Japanese patent No. 980 3671 (1998) describes the production by fermentation of DHA and another PUFA, docosapentaenoic acid (DPA) from lipids of thraustochytrid fungi. US Patent No. 5,340,594 describes a process for production of whole-celled or extracted microbial products using thraustochytrid fungi with a high concentration of the omega-3. PUFAs. US Patent No. 5,340,742 discloses a process for growing the thraustochytrid fungi in defined media suitable for their growth. All the above patents relate to screening numerous thraustochytrid cultures, selecting the strain with the highest DHA and EPA content, prepare mutant strains of these and cultivate such strains under optimal culture conditions for commercial application.

The present invention aims to further increase the DHA and EPA levels in thraustochytrid fungi so that they will provide still higher commercial yields of the said PUFAs. Besides, the above mentioned prior art patents reject a large number of strains of thraustochytrid fungi, which might have only moderate DHA and EPA concentrations. Strains of thraustochytrid fungi with moderate amounts of DHA and EPA can be made to produce large amounts of these PUFAs by growing them in a medium with increased viscosity. Strains of thraustochytrid fungi that naturally have high concentrations of DHA and EPA can be made to produce even more.

### OBJECTS OF THE INVENTION

The main object of the present invention is to enhance the amounts of PUFAs in thraustochytrid fungi, which obviates the drawbacks as detailed above.

The object of the invention is also to make strains of thraustochytrids to produce higher amounts of DHA and EPA than they normally produce using optimal nutrient conditions.

The object of the present invention is also to enhance the levels of these fatty acids by growing the cultures of thraustochytrid fungi in a medium with increased viscosity.

### SUMMARY OF THE INVENTION

To meet the above objects, the present invention provides a method for enhancing levels of polyunsaturated fatty acid levels in thraustochytrid fungi, using culture media supplemented with polyvinyl pyrrolidone (PVP) to increase viscosity.

### DETAILED DESCRIPTION OF THE INVENTION

Accordingly, the present invention provides a method for enhancing levels of polyunsaturated fatty acid levels in thraustochytrid fungi, using culture media supplemented with polyvinyl pyrrolidone (PVP) to increase viscosity and which comprises : Step a: Providing a thraustochytrid fungus strain NIO-TH 21, corresponding to the species *Ulkenia radiata* Gaertner, being the culture with Accession No.AB22115 of the National Institute of Bioscience and Human-Technology, Japan; Step b: Inoculating the above said strain in a culture medium; Step c: Growing the culture for 2 days at a temperature ranging from 25 to 30°C; Step d: Obtaining the cultures for use as inoculum using the above said medium to inoculate a medium with different concentrations of polyvinyl pyrrolidone (PVP); Step e: Growing the culture separately for 2 to 5 days at a temperature ranging from 25 to 30°C; Step f: Harvesting the cells from the above culture by centrifugation and extracting the enhanced amounts of docosahexaenoic acid (DHA) and eicosapentaenoic acids (EPA).

The viscosity of the medium may be increased by incorporating polyvinyl pyrrolidone (PVP) at concentrations ranging from 0:1 to 1:0%.

In the present invention, a process is provided to enhance the levels of the PUFAs in cells of thraustochytrid fungi.

The PUFAs that are enhanced are DHA and EPA.

DHA and EPA are enhanced in cells of thraustochytrid fungi by growing the cultures in a medium with increased viscosity.

The increase in viscosity may be provided by incorporating a substance that is not utilized as nutrients such as polyvinyl pyrrolidone (PVP) at concentrations ranging from 0.1 to 1.0%.

The culture medium used may comprise peptone in the range of 0.5% Wt. to 1.5% Wt, preferably, 1.5% Wt.; Yeast extract in the range of 0.01% Wt. to 0.1% Wt., preferably, 0.1% Wt.; Glucose in the range of 0.01% to 1.0 % Wt., preferably, 1.0% Wt; and Sea water of 100 ml.

The culture medium may comprise 1.5% peptone; 0.1 % yeast extract; 1.0% glucose; 0.5% polyvinyl pyrrolidone and 100 ml sea water.

According to the present invention, culture of the candidate species of the thrausochytrid fungus, which contains the omega-3 PUFAs DHA and EPA is first inoculated into a liquid nutrient medium. A suitable medium for example, is one containing peptone, yeast extract, glucose and sea water. Any other medium that supports good growth of the fungus also may be used. The culture is grown for 2 days at a room temperature ranging from 25 to 30°C. This culture is used as the inoculum and used to inoculate a medium with enhanced viscosity. The compound that is added to increase viscosity may be one of the common polymers, such as dextran or polyvinyl pyrrolidone (PVP) that are not utilised as nutrients by the organisms, but only contribute to increasing the medium viscosity. For example, polyvinyl pyrrolidone (PVP) is a water-soluble polymer of basic nature (McGraw-Hill Encyclopaedia of Science and Technology, Vol. 10, 1982). PVP is commonly used to increase fluid viscosity and is a suitable agent for this purpose (Podolsky, R.D. and RB. Emlet, 1993. Journal of experimental biology 176: 207-221). In the present example, PVP at concentrations of 0.1 to 1.0 % are added to the medium. Cultures may be grown in flasks on a rotary shaker in the laboratory or in a fermentor when large scale cultivation is required. The culture is allowed to grow at room temperature of 25 to 30°C or any temperature at which the particular strain grows best. After a suitable period, for example 2 to 7 days growth, cells from the culture are harvested. This may be done by any appropriate method, such as centrifugation, continuous flow centrifugation, filtration etc. Cells thus obtained may be used for all applications that require thraustochytrid cells. Such use may include cell biomass for animal feed, human food supplement or extraction of pure DHA and EPA.

The present invention thus relates to a process to enhance the levels of the omega-3 PUFAs, DHA and EPA. By this process, strains of cultures of thraustochytrids can be made to produce higher levels of these PUFAs than they do under other conditions. Besides, even strains that contain only moderate quantities of these PUFAs under normal conditions can be made to produce greater amounts within their cells.

The invention is described in detail hereafter with reference to the accompanying drawings, which are provided merely to illustrate the invention.

### BRIEF DESCRIPTION OF THE ACCOMPANYING DRAWINGS

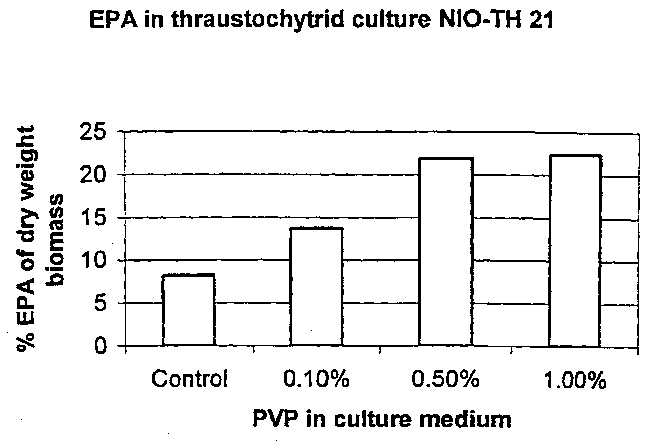

Figure 1 represents the EPA contents of a thraustochytrid strain NIO-TH 21, corresponding in its morphology and life cycle to the species *Ulkenia radiata* Gaertner, being the culture with Accession No. AB22115 of the National Institute of Bioscience and Human-Technology, Japan, when grown in a liquid nutrient culture medium.

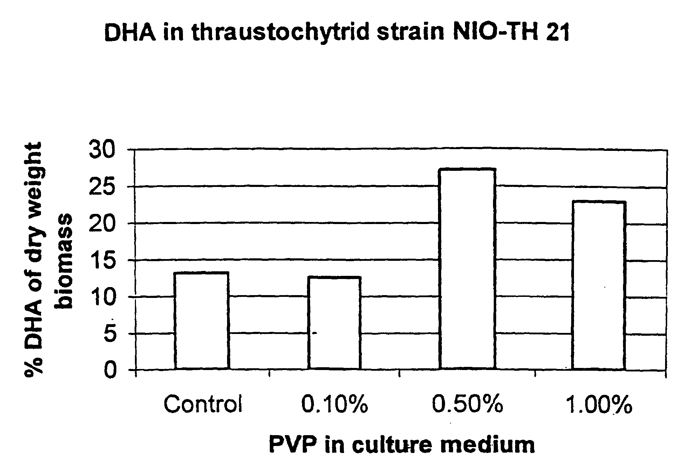

Figure 2 represents the DHA contents of a thraustochytrid strain NIO-TH 21, corresponding in its morphology and life cycle to the species *Ulkenia radiata* Gaertner, being the culture with Accession No. AB22115 of the National Institute of Bioscience and Human-Technology, Japan, when grown in a liquid nutrient culture medium.

The following examples are given by way of illustrations of the present invention and therefore, should not be construed to limit the scope of the present invention.

### EXAMPLE - 1

A culture of a thraustochytrid, belonging to strain # NIO-TH 21 was inoculated into 100 ml of a culture medium containing: gelatin peptone - 1.5% Wt.; Yeast extract - 0.1 % Wt.; Glucose - 1.0% Wt. and Sea water - 100 ml. The cultures were grown for 2 days on a shaker at room temperature of 25 - 30°C. These cultures were used as innoculum for the experiment. Three sets of cultures were set up using a medium with the same composition as above. The first set contained an addition of 0.1 % polyvinyl pyrrolidone. The second set contained 0.5 % PVP, while the third contained 1.0 % PVP. The experiment was carried out by adding 10 ml of the innoculum into 100 ml of the culture medium for each set. The cultures were grown for 3 days on a shaker at room temperature of 25 - 30°C. At the end of this period, cells were harvested by centrifugation, fatty acids extracted and analyzed by gas chromatography. Cultures grown in media with increased viscosity by adding PVP contained nearly 0.5 to 2 times more EPA than those grown in a medium without increased PVP (Fig. 1).

### EXAMPLE - 2

A culture of a thraustochytrid, belonging to strain # NIO-TH21 was inoculated into 100 ml of a culture medium containing: gelatin peptone - 1.5% Wt.; Yeast extract - 0.1 % Wt.; Glucose - 1.0% Wt. and sea water - 100 ml. The cultures were grown for 2 days on a shaker at room temperature of 25 - 30°C. These cultures were used as innoculum for the experiment. Three sets of cultures were set up using a medium with the same composition as above. The first set contained an addition of 0.1 % polyvinyl pyrrolidone. The second set contained 0.5 % PVP, while the third contained 1.0 % PVP. The experiment was carried out by adding 10 ml of the innoculum into 100 ml of the culture medium for each set. The cultures were grown for 3 days on a shaker at room temperature of 25-30°C. At the end of this period, cells were harvested by centrifugation, fatty acids extracted and analyzed by gas chromatography. Cultures grown in media with increased viscosity by adding 0.5 and 1.0 % PVP contained nearly 2 times more DHA than those grown in a medium without PVP (Fig. 1).
The main advantages of the present invention are:
1. The DHA and EPA levels of thraustochytrids normally present in cultures can be further enhanced.
2. Even those strains that have only moderate quantities of DHA and EPA can be enriched in these fatty acids.
3. The viscosity of the medium is increased by addition of polyvinyl pyrrolidone, an easily available chemical.
4. Polyvinyl pyrrolidone is not used as nutrition by the cultures and, therefore, does not interfere with their normal metabolism.
5. Polyvinyl pyrrolidone is not toxic to the cultures and does not harm their normal metabolism.

## Claims

1. A method for enhancing levels of polyunsaturated fatty acid levels in thraustochytrid fungi, using media supplemented with polyvinyl pyrrolidone (PVP) to increase viscosity and which comprises:
(a) providing a thraustochytrid fungus strain NIO-TH 21, corresponding to the species *Ulkenia radiata* Gaertner, being the culture with Accession No. AB22115 of the National Institute of Bioscience and Human-Technology, Japan;
(b) inoculating the above said strain in a culture medium;
(c) growing the culture for 2 days at a temperature ranging from 25 to 30°C;
(d) obtaining the cultures for use as innoculum using the above said medium to inoculate a medium with different concentrations of polyvinyl pyrrolidone (PVP);
(e) growing the culture separately for 2 to 5 days at a temperature ranging from 25 to 30°C; and
(f) harvesting the cells from the above culture by centrifugation and extracting the enhanced amounts of docosahexaenoic acid (DHA) and eicosapentaenoic acids (EPA).

2. A method as claimed in claim 1 wherein the culture medium used comprising peptone in the range of 0.5% Wt. to 1.5% Wt., preferably, 1.5% Wt.; Yeast extract in the range of 0.01% Wt. to 0.1% Wt., preferably, 0.1% Wt.; Glucose in the range of 0.01% to 1.0 % Wt., preferably, 1.0% Wt.; and Sea water of 100 ml.

3. A method as claimed in Claim 1 wherein the culture medium comprises 1.5% peptone; 0.1% yeast extract; 1.0% glucose; 0.5% polyvinyl pyrrolidone and 100 ml sea water.

4. A method as claimed in claim 1 wherein the increase in viscosity is provided by incorporating a substance that is not utilized as nutrients such as polyvinyl pyrrolidone (PVP) at concentrations ranging from 0.1 to 1.0% by Wt.

## Patentansprüche

1. Verfahren zur Steigerung des Spiegels von mehrfach ungesättigten Fettsäuren in Thraustochytrit Pilzen, unter Anwendung von mit Polyvinylpyrrolidon (PVP) angereicherten Medien um die Viskosität zu erhöhen, mit folgenden Schritten :
a) Bereitstellen eines Thraustochytrit Pilz Stammes NIO-TH 21, der der Spezies *Ulkenia radiata* Gaertner, bzw. der Kultur mit der Hinterlegungsnummer AB22115 des National Institutes for Bioscience and Human Technology, Japan entspricht ;
b) Inokulieren des oben bezeichneten Stammes in ein Kulturmedium ;
c) Wachsen der Kultur während 2 Tagen bei einer Temperatur zwischen 25°C and 30°C;
d) Gewinnung der Kulturen für die Verwendung als Innokulum unter Verwendung des oben genannten Mediums um ein Medium mit unterschiedlichen Konzentrationen an Polyvinylpyrrolidon (PVP) zu inokulieren ;
e) Getrenntes Wachsen der Kultur während 2 bis 5 Tagen bei einer Temperatur zwischen 25°C and 30°C ; und
f) Ernten der Zellen der obengenannten Kultur durch Zentrifugieren und Abziehen der gesteigerten Mengen an Dokosahexaesäure (DHA) und Eikosapentansäure (EPA).

2. Verfahren nach Anspruch 1, in dem das verwendete Kulturmedium von 0.5 Gew.% bis 1.5 Gew.%, vorzugsweise 1.5 Gew.% Peptone; 0.01 Gew.% bis 0.1 Gew. %, vorzugsweise 0.1 Gew.% Hefeextrakt; 0.01 Gew.% bis 1.0 Gew. %, vorzugsweise 1.0 Gew.% Glykose, und 100 ml Meerwasser enthält.

3. Verfahren nach Anspruch 1, in dem das Kulturmedium 1.5% Peptone ; 0.1% Hefeextrakt ; 1.0% Glykose ; 0.5% Polyvinylpyrrolidon und 10 ml Meerwasser enthält.

4. Verfahren nach Anspruch 1, in dem die Zunahme an Viskosität durch die Eingliederung einer Substanz vorgenommen wird, welche nicht als Nährmittel, wie beispielsweise Polyvinylpyrrolidon (PVP) verwendet wird, in einer Konzentration zwischen 0.1 % und 1.0 Gew.%.

## Revendications

1. Procédé pour accroître les niveaux des taux d'acides gras polyinsaturés dans les champignons thraustochytrides, qui utilise des milieux supplémentés par de la polyvinylpyrrolidone (PVP) pour augmenter la viscosité, et qui comprend les étapes consistant :
(a) à mettre en oeuvre une souche de champignon thraustochytride NIO-TH 21, correspondant à l'espèce *Ulkenia radiara* Gaertner, qui est la culture avec le Numéro Matricule AB22 115 du National Institute of Bioscience and Human Technology, Japon ;
(b) à inoculer ladite souche ci-dessus dans un milieu de culture ;
(c) à faire pousser la culture pendant 2 jours à une température allant de 25 à 30 °C ;
(d) à obtenir les cultures pour une utilisation en tant qu'inoculum, en utilisant ledit milieu ci-dessus pour inoculer un milieu avec différentes concentrations de polyvinylpyrrolidone (PVP) ;
(e) à faire pousser la culture séparément pendant 2 à 5 jours à une température allant de 25 à 30 °C ; et
(f) à récolter les cellules à partir de la culture ci-dessus par centrifugation, et à extraire les quantités accrues d'acide docosahexaénoïque (ADH) et d'acide eicosapentaénoïque (AEP).

2. Procédé selon la revendication 1, dans lequel le milieu de culture utilisé comprend de la peptone dans la gamme de 0,5 % en poids à 1,5 % en poids, de préférence dans une proportion de 1,5 % en poids ; de l'extrait de levure dans la gamme de 0,01 % en poids à 0,1 % en poids, de préférence dans une proportion de 0,1 % en poids ; du glucose dans la gamme de 0,01 % à 1,0 % en poids, de préférence dans une proportion de 1,0 % en poids ; et 100 mL d'eau de mer.

3. Procédé selon la revendication 1, dans lequel le milieu de culture comprend 1,5 % de peptone ; 0,1% d'extrait de levure; 1,0 % de glucose; 0,5% de polyvinylpyrrolidone et 100 mL d'eau de mer.

4. Procédé selon la revendication 1, dans lequel l'augmentation de la viscosité est fournie par l'incorporation d'une substance qui n'est pas utilisée en tant que nutriment, telle que la polyvinylpyrrolidone (PVP) à des concentrations allant de 0,1 à 1,0 % en poids.
